# EUROPÄISCHE PATENTSCHRIFT

(11) **EP 1 687 857 B1**
(45) Veröffentlichungstag und Bekanntmachung des Hinweises auf die Patenterteilung: **09.09.2009**
(21) Anmeldenummer: 04803245.2
(22) Anmeldetag: 24.11.2004
(51) Int. Cl.: H01L 51/30, C07D 251/24, C07D 253/06, C07D 239/26

(54) **ORGANISCHES ELEKTROLUMINESZENZELEMENT**
ORGANIC ELECTROLUMINESCENT ELEMENT
ELEMENT ORGANIQUE ELECTROLUMINESCENT

(30) Priorität: 27.11.2003 DE 10356099
(43) Veröffentlichungstag der Anmeldung: 09.08.2006
(73) Patentinhaber: Merck Patent GmbH, 64293 Darmstadt (DE)
(72) Erfinder: VESTWEBER, Horst, 34330 Gilserberg-Winterscheid (DE); GERHARD, Anja, 97209 Veitschöchheim (DE); STÖSSEL, Philipp, 60487 Frankfurt am Main (DE)
(86) Internationale Anmeldenummer: PCT/EP2004/013314
(87) Internationale Veröffentlichungsnummer: WO 2005/053055

(56) Entgegenhaltungen:
- EP-A- 1 385 221
- WO-A-20/04077885
- DE-A1- 4 446 818
- US-A1- 2002 034 659
- US-B1- 6 229 012
- US-B1- 6 352 791
- WU C C ET AL: "Highly bright blue organic light-emitting devices using spirobifluorene-cored conjugated compounds" APPLIED PHYSICS LETTERS, AMERICAN INSTITUTE OF PHYSICS. NEW YORK, US, Bd. 81, Nr. 4, 22. Juli 2002 (2002-07-22), Seiten 577-579, XP012033022 ISSN: 0003-6951
- FOURNIER, JEAN-HUGUES ET AL: "Molecular Tectonics. Porous Hydrogen-Bonded Networks Built from Derivatives of 9,9'-Spirobifluorene" JOURNAL OF ORGANIC CHEMISTRY ( 2004 ), 69(6), 1762-1775 CODEN: JOCEAH; ISSN: 0022-3263, 13. September 2003 (2003-09-13), XP002322041
- PATENT ABSTRACTS OF JAPAN Bd. 2003, Nr. 07, 3. Juli 2003 (2003-07-03) -& JP 2003 086381 A (TORAY IND INC), 20. März 2003 (2003-03-20)

## Beschreibung

Organische und metallorganische Verbindungen finden Einsatz als funktionelle Materialien in einer Reihe verschiedenartiger Anwendungen, die im weitesten Sinne der Elektronikindustrie zugerechnet werden können. Bei den auf organischen Komponenten basierenden organischen Elektrolumineszenzvorrichtungen (allg. Beschreibung des Aufbaus vgl. US 4,539,507 und US 5,151,629) bzw. deren Einzelbauteilen, den organischen lichtemittierenden Dioden (OLEDs), ist die Markteinführung bereits erfolgt, wie die Autoradios mit "organischem Display" der Firma Pioneer oder eine Digitalkamera der Firma Kodak belegen. Weitere derartige Produkte stehen kurz vor der Einführung. Dennoch sind hier noch deutliche Verbesserungen nötig, um diese Displays zu einer echten Konkurrenz zu den derzeit marktbeherrschenden Flüssigkristallanzeigen (LCD) zu machen bzw. diese zu überflügeln.

Eine Entwicklung, die sich in den letzten Jahren abzeichnet, ist der Einsatz metallorganischer Komplexe, die Phosphoreszenz statt Fluoreszenz zeigen (M. A. Baldo et al., Appl. Phys. Lett. 1999, 75, 4-6). Aus theoretischen Spin-statistischen Gründen ist unter Verwendung metallorganischer Verbindungen als Phosphoreszenz-Emitter eine bis zu vierfache Energie- und Leistungseffizienz möglich. Ob sich diese neue Entwicklung durchsetzen wird, hängt davon ab, ob entsprechende Device-Kompositionen gefunden werden können, die diese Vorteile (Triplett-Emission = Phosphoreszenz gegenüber Singulett-Emission = Fluoreszenz) auch in den OLEDs umsetzen können. Hier ist nicht nur die Entwicklung der metallorganischen Verbindungen selbst von Bedeutung, sondern vor allem auch von weiteren speziell hierfür benötigten Materialien, wie beispielsweise Matrix- oder Lochblockiermaterialien.

Üblicherweise besteht eine organische Elektrolumineszenzvorrichtung aus mehreren Schichten, die mittels Vakuummethoden oder unterschiedlicher Drucktechniken aufeinander aufgebracht werden. Für phosphoreszierende organische Elektrolumineszenzvorrichungen sind diese Schichten im Einzelnen:
1. Trägerplatte = Substrat (üblicherweise Glas oder Kunststofffolie);
2. Transparente Anode (üblicherweise Indium-Zinn-Oxid, ITO);
3. Lochinjektionsschicht (**H**ole **I**njection **L**ayer = HIL): z. B. auf der Basis von Kupferphthalocyanin (CuPc) oder leitfähigen Polymeren;
4. Lochtransportschicht(en) (**H**ole **T**ransport **L**ayer = HTL): üblicherweise auf Basis von Triarylaminderivaten, z. B. 4,4',4"-Tris(N-1-naphthyl-N-phenyl-amino)-triphenylamin (NaphDATA) als erste Schicht und N,N'-Di-(1-naphthyl)-N,N'-diphenylbenzidin (NPB) als zweite Schicht;
5. Emissionsschicht(en) (**Em**ission **L**ayer = EML): üblicherweise bei phosphoreszierenden Devices aus einem Matrixmaterial, z. B. 4,4'-Bis(carbazol-9-yl)-biphenyl (CBP), das mit einem Phosphoreszenzfarbstoff, z. B. Tris(phenylpyridyl)-iridium (Ir(PPy)₃) oder Tris(2-benzothiophenylpyridyl)-iridium (Ir(BTP)₃), dotiert ist;
6. Lochblockierschicht (**H**ole **B**locking **L**ayer = HBL): üblicherweise aus BCP (2,9-Dimethyl-4,7-diphenyl-1,10-phenanthrolin = Bathocuproin) oder Bis-(2-methyl-8-hydroxychinolinato)-(4-phenylphenolato)-aluminium(III) (BAlq);
7. Elektronentransportschicht (**E**lectron **T**ransport **L**ayer = ETL): meist auf Basis von Aluminium-tris-8-hydroxychinolinat (AlQ₃);
8. Elektroneninjektionsschicht (**E**lectron **I**njection **L**ayer = EIL, auch Isolatorschicht = ISL genannt): dünne Schicht aus einem Material mit einer hohen Dielektrizitätskonstanten, wie z. B. LiF, Li₂O, BaF₂, MgO, NaF;
9. Kathode: in der Regel Metalle, Metallkombinationen oder Metalllegierungen mit niedriger Austrittsarbeit, z. B. Ca, Ba, Cs, Mg, Al, In, Mg/Ag.

Je nach Deviceaufbau können auch mehrere dieser Schichten zusammenfallen, bzw. es muss nicht notwendigerweise jede dieser Schichten vorhanden sein.

Allerdings gibt es immer noch erhebliche Probleme, die einer dringenden Verbesserung bedürfen, um hochwertige Vollfarbanwendungen zu ermöglichen:
1. So ist v. a. die operative Lebensdauer von OLEDs immer noch zu gering, so dass bislang nur einfache Anwendungen kommerziell realisiert werden konnten.
2. Aus der kurzen Lebensdauer ergibt sich ein Folgeproblem: Gerade für VollfarbAnwendungen ("full-colour displays") ist es besonders schlecht, wenn die einzelnen Farben unterschiedlich schnell altern, wie es derzeit der Fall ist. Dies führt dazu, dass sich schon vor Ende der Lebensdauer (die in der Regel durch einen Abfall auf 50 % der Anfangshelligkeit definiert ist) der Weißpunkt deutlich verschiebt, d. h. die Farbtreue der Darstellung im Display wird schlechter. Um dies zu umgehen, definieren einige Displayhersteller die Lebensdauer als 70 % oder 90 % Lebensdauer (d. h. Abfall der Anfangshelligkeit auf 70 % bzw. auf 90 % des Anfangswerts). Dies führt jedoch dazu, dass die Lebensdauer noch kürzer wird.
3. Die Alterungsprozesse gehen i. d. R. mit einem Anstieg der Spannung einher. Dieser Effekt macht spannungsgetriebene organische Elektrolumineszenzvorrichtungen schwierig bzw. unmöglich. Eine stromgetriebene Ansteuerung ist aber in diesem Fall aufwändiger und teurer.
4. Die benötigte Betriebsspannung ist gerade bei effizienten phosphoreszierenden OLEDs recht hoch und muss daher verringert werden, um die Leistungseffizienz zu verbessern.
5. Die Effizienz, insbesondere die Leistungseffizienz (gemessen in Im/W), von phosphoreszierenden OLEDs ist zwar akzeptabel, aber auch hier sind immer noch Verbesserungen erwünscht.
6. Der Aufbau der OLEDs ist durch die Vielzahl organischer Schichten komplex und technologisch aufwändig; eine Reduktion der Schichtenanzahl ist für die Produktion wünschenswert, um die Anzahl der Produktionsschritte zu verringern, dadurch die Technologie zu vereinfachen und die Produktionssicherheit zu erhöhen.

Die oben genannten Gründe machen Verbesserungen bei der Herstellung von OLEDs notwendig.

Bei phosphoreszierenden OLEDs wird üblicherweise eine Lochblockierschicht (HBL) folgend auf die Emitterschicht zur Steigerung der Effizienz und Lebensdauer verwendet. Diese Devicestrukturen werden meist nach dem Kriterium der maximalen Effizienz optimiert. Dabei kommt häufig BCP (Bathocuproin) als Lochblockiermaterial (HBM) zum Einsatz, womit sehr gute Effizienzen erzielt werden (D. F. O'Brien et al., Appl. Phys. Lett. 1999, 74, 442), allerdings mit dem entscheidenden Nachteil, dass die Lebensdauer der OLEDs mit BCP stark eingeschränkt ist. T. Tsutsui et al. (Japanese J. Appl. Phys. 1999, 38, L1502) geben als Grund für die schlechte Lebensdauer die geringe Stabilität von BCP an, so dass diese Devices nicht in hochwertigen Displays Verwendung finden können. Ein weiteres Lochblockiermaterial ist Bis-(2-methyl-8-hydroxychinolato)-(4-phenylphenolato)-aluminium(III) (BAlq). Damit konnte die Stabilität und Lebensdauer der Devices deutlich verbessert werden, allerdings mit dem Nebeneffekt, dass die Quanteneffizienz der Devices mit BAlq ca. 40 % niedriger ist als mit BCP (T. Watanabe et al., Proc. SPIE 2001, 4105, 175). Kwong et al. (Appl. Phys. Lett. 2002, 81, 162) erzielten damit Lebensdauern von 10000 h bei 100 cd/m² mit Tris(phenylpyridyl)iridium(III). Allerdings zeigte dieses Device nur eine Effizienz von 19 cd/A, was weit hinter dem Stand der Technik zurückliegt. Somit sind mit BAlq zwar gute Lebensdauern möglich, insgesamt ist es jedoch kein zufriedenstellendes Lochblockiermaterial, da die erreichte Effizienz zu niedrig ist.

Aus dieser Beschreibung geht klar hervor, dass die bislang verwendeten Lochblockiermaterialien (HBM), wie z. B. BCP oder BAlq, zu unbefriedigenden Nebeneffekten führen. Es besteht also weiterhin ein Bedarf an Lochblockiermaterialien, die in OLEDs zu guten Effizienzen führen, gleichzeitig aber auch zu hohen Lebensdauern. Es wurde nun überraschend gefunden, dass OLEDs, die bestimmte - im Folgenden aufgeführte - Heterocyclen, insbesondere Diazine und Triazine, als Lochblockiermaterialien enthalten, deutliche Verbesserungen gegenüber dem Stand der Technik aufweisen. Mit diesen Lochblockiermaterialien ist es möglich, gleichzeitig hohe Effizienzen und gute Lebensdauern zu erhalten, was mit Materialien gemäß dem Stand der Technik nicht möglich ist. Zudem wurde gefunden, dass mit den neuen Lochblockiermaterialien nicht notwendigerweise eine separate Elektronentransportschicht verwendet werden muss, was einen technologischen Vorteil darstellt, und dass dadurch zusätzlich die Betriebsspannungen deutlich gesenkt werden können, was einer höheren Leistungseffizienz entspricht.

Die Verwendung von Triazinen, Pyrimidinen, Pyrazinen und Pyridazinen in organischen Elektrolumineszenzvorrichtungen als Emissions- oder Ladungstransportmaterial ist in der Literatur bereits beschrieben. Diese Materialien wurden als fluoreszierende Emitter bzw. in Kombination mit fluoreszierenden Emittern beschrieben. US 6352791 und US 6225467 beschreiben Triazine, insbesondere speziell substituierte Triazine, als Elektronentransportmaterialien in OLEDs. A. Bacher et al. (Inorg. and Org. Electroluminescence (Int. Workshop on Electroluminescence) 1996, 109-112) berichten eine dreifach höhere Effizienz, wenn zwischen AlQ₃ als Emitter und die Kathode Triazinderivate als Elektronentransportschicht eingebracht werden. Über die Lebensdauer wird keine Aussage gemacht. Pyrazine als Elektronentransportmaterialien werden beispielsweise beschrieben von T. Oyamada et al. (Chem. Lett. 2003, 32, 388). Andererseits können substituierte Pyrimidin- und Triazinderivate auch als Lochtransportmaterialien in OLEDs eingesetzt werden (US 5716722). In JP 2002/212170 werden Triazinderivate als elektrofluoreszierdende Verbindungen beschrieben, ebenso von J. Pang et al. (J. Mater. Chem. 2002, 12, 206).
In JP 2003/282270 werden Phenylpyridin-Derivate in OLEDs beschrieben. Diese können, außer etlichen anderen Gruppen, auch Triazin, Pyrimidin, Pyrazin oder Pyridazin enthalten. Jedoch wird der positive Effekt dieser Verbindungen auf die Phenylpyridin-Einheiten zurückgeführt, und nicht auf das Triazin, Pyrimidin, Pyrazin oder Pyridazin, so dass diese Anmeldung als zufällige Offenbarung zu bewerten ist.

C. C. Wu et al. (Appl. Phys. Lett. 2002, 81, 577-579) offenbaren die Verwendung von einem Spirobifluorenderivat, welches mit Pyrimidingruppen substituiert ist, als blau emittierende Verbindung bzw. als emittierenden Host in einer organischen Elektrolumineszenzvorrichtung.

Aus dem oben zitierten Stand der Technik ist nicht ersichtlich, wie Diazin- und Triazinderivate sinnvoll in phosphoreszierenden OLEDs angewendet werden könnten, da diese Materialien sowohl als Elektronenleiter, wie auch als Lochleiter oder als (fluoreszierende) Emitter beschrieben sind. Insbesondere ist in der Literatur von führenden Fachleuten auf dem Gebiet der phosphoreszierenden OLEDs beschrieben (R. C. Kwong, M. E. Thompson, S. R. Forrest et al., Appl. Phys. Lett. 2002, 81, 162), dass elektronenarme Heterocyclen, wie beispielsweise Triazine, als Lochblockiermaterialien in phosphoreszierenden OLEDs zu sehr schlechten Lebensdauern, typischerweise im Bereich von weniger als 100 h bei Helligkeiten von 500 cd/m² führen, was weit hinter dem Stand der Technik zurückliegt. Daraus hätte ein Fachmann ableiten können, dass diese Kombination also ungeeignet ist, um eine technische Verbesserung zu erreichen. Dies lässt keineswegs vermuten, dass damit gute Ergebnisse erzielt werden können.

Weiterhin ist aus J.-H. Fournier et al., J. Org. Chem. 2004, 69, 1762-1775 ein Spirobifluorenderivat bekannt, welches in 3,3',6,6'-Position mit 2,4-Diamino-1,3,5-triazin substituiert ist. Diese Verbindung wird als Baustein für poröse Netzwerke mit Wasserstoffbrückenbindungen beschrieben. Eine Verwendung dieser Verbindungen in organischen Elektrolumineszenzvorrichtungen ist nicht offenbart.

Gegenstand der Erfindung ist eine organische Elektrolumineszenzvorrichtung, enthaltend eine Anode, eine Kathode und eine Emissionsschicht, bestehend aus mindestens einem Matrixmaterial, welches mit mindestens einem phosphoreszierenden Emitter dotiert ist, **dadurch gekennzeichnet, dass** zwischen die Emissionsschicht und die Kathode eine Lochblockierschicht eingebracht ist, die eine Verbindung gemäß Formel (1) enthält, 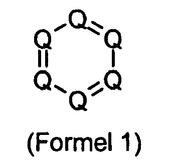 wobei für die verwendeten Symbole und Indizes gilt:
- Q: ist bei jedem Auftreten gleich oder verschieden N oder CR mit der Maßgabe, dass mindestens zwei und maximal vier Q für Stickstoff stehen;
- R: ist bei jedem Auftreten gleich oder verschieden H, NO₂, CN, N(R¹)₂, eine geradkettige, verzweigte oder cyclische Alkyl- oder Alkoxygruppe mit 1 bis 40 C-Atomen, wobei eine oder mehrere nicht benachbarte CH₂-Gruppen durch -R¹C=CR¹-, -C≡C-, Si(R¹)₂, Ge(R¹)₂, Sn(R¹)₂, -O-, -S- oder -NR¹- ersetzt sein können und wobei ein oder mehrere H-Atome durch F oder eine aromatische Gruppe R¹ ersetzt sein können, oder
ein aromatisches bzw. heteroaromatisches Ringsystem oder eine Aryloxy- oder Heteroaryloxygruppe jeweils mit 1 bis 40 aromatischen C-Atomen, wobei ein oder mehrere H-Atome durch F, Cl, Br oder I ersetzt sein können oder die durch einen oder mehrere nicht-aromatische Reste R substituiert sein kann; dabei können mehrere Substituenten R ein weiteres mono- oder
polycyclisches, aliphatisches oder aromatisches Ringsystem aufspannen, oder ein über eine bivalente Gruppe -Z- gebundenes aromatisches bzw. heteroaromatisches Ringsystem oder eine Aryloxy- oder Heteroaryloxygruppe jeweils mit 1 bis 40 aromatischen C-Atomen, wobei ein oder mehrere H-Atome durch F, Cl, Br oder I ersetzt sein können oder die durch einen oder mehrere nicht aromatische Reste R substituiert sein kann; dabei können mehrere Substituenten R ein weiteres mono- oder polycyclisches, aliphatisches oder aromatisches Ringsystem aufspannen;
- R¹: ist bei jedem Auftreten gleich oder verschieden H oder ein aliphatischer, aromatischer oder heteroaromatischer Kohlenwasserstoffrest mit 1 bis 20 C-Atomen, wobei mehrere Substituenten R¹ bzw. R¹ mit R auch ein weiteres mono- oder polycyclisches, aliphatisches oder aromatisches Ringsystem aufspannen können;
- Z: ist bei jedem Auftreten gleich oder verschieden ein geradkettiger, verzweigter oder cyclischer, bevorzugt konjugierter Rest mit 1 bis 40 C-Atomen, der bevorzugt in Konjugation mit den beiden anderen Substituenten steht, wobei bevorzugt die Anzahl der Atome von Z, die die Gruppe gemäß Formel (1) und den aromatischen Rest verknüpfen, eine gerade Zahl beträgt, wobei ein oder mehrere nicht benachbarte C-Atome durch -O-, -S- oder -NR¹- ersetzt sein können oder ein oder mehrere C-Atome durch einen Rest R¹ oder Halogen substituiert sein können;
mit der Maßgabe, dass R kein substituiertes oder unsubstituiertes Phenylpyridin enthält;
**dadurch gekennzeichnet, dass** in mindestens einem der Reste R ein 9,9'-Spirobifluorenderivat, ein 9,9-disubstituiertes Fluorenderivat, ein 6,6- und/oder 12,12-di- oder tetrasubstituiertes Indenofluorenderivat, ein Tetraarylmethanderivat oder ein Triptycenderivat enthalten ist.

Bevorzugt weist die Verbindung gemäß Formel (1) ein Molekulargewicht von mindestens 350 g/mol auf.

Unter einem aromatischen bzw. heteroaromatischen Ringsystem im Sinne dieser Erfindung soll ein System verstanden werden, das nicht notwendigerweise nur Arylgruppen bzw. Heteroarylgruppen enthält, sondern in dem auch mehrere Aryl- bzw. Heteroarylgruppen durch eine kurze nicht-aromatische Einheit, wie beispielsweise sp³-hybridisierter C, O, N, etc. unterbrochen sein können. So sollen also beispielsweise auch Systeme wie 9,9'-Spirobifluoren, 9,9-Diarylfluoren, Triarylamin, Diarylether, etc. als aromatische Systeme verstanden werden.

Die OLED kann noch weitere Schichten enthalten, wie zum Beispiel Lochinjektionsschicht, Lochtransportschicht, Elektroneninjektionsschicht und/oder Elektronentransportschicht. Es sei allerdings darauf verwiesen, dass nicht notwendigerweise alle diese Schichten vorhanden sein müssen. So wurde gefunden, dass erfindungsgemäße OLEDs, die in der Lochblockierschicht Verbindungen gemäß Formel (1) enthalten, weiterhin vergleichbar gute Effizienzen und Lebensdauern bei verringerter Betriebsspannung liefern, wenn keine separaten Elektroneninjektions- und Elektronentransportschichten verwendet werden. Bevorzugt enthält die erfindungsgemäße Lochblockierschicht mindestens 50 % Verbindungen gemäß Formel (1), besonders bevorzugt mindestens 80 %, ganz besonders bevorzugt besteht diese nur aus Verbindungen gemäß Formel (1).

Bevorzugte Strukturen gemäß Formel (1) enthalten im Ring zwei oder drei Stickstoffatome. Es handelt sich dabei Diazine oder Triazine, also um Pyridazine (1,2-Diazine), Pyrimidine (1,3-Diazine), Pyrazine (1,4-Diazine), 1,2,3-, 1,2,4- oder 1,3,5-Triazine. Besonders bevorzugt sind Pyrimidine oder Triazine, insbesondere 1,2,4- und 1,3,5-Triazine.

Auch wenn dies aus der Beschreibung hervorgeht, sei hier ausdrücklich darauf hingewiesen, dass das Lochblockiermaterial auch mehr als eine solche Diazin- oder Triazineinheit enthalten kann.

Als besonders geeignete Lochblockiermaterialien haben sich Verbindungen erwiesen, die nicht planar aufgebaut sind. Während der Grundkörper (also der Sechsring des Diazins bzw. Triazins) immer planar aufgebaut ist, können entsprechende Substituenten (enthalten in R) für eine Abweichung der Gesamtstruktur von der Planarität sorgen. Dies ist insbesondere dann der Fall, wenn mindestens einer der Substituenten R ein sp³-hybridisiertes Kohlenstoffatom (oder entsprechend auch Silicium, Germanium, Stickstoff, etc.) enthält, das dadurch näherungsweise tetraedrische (oder im Fall von Stickstoff pyramidale) Geometrie aufweist.
Bevorzugte Lochblockiermaterialien sind daher Verbindungen gemäß Formel (1), in denen mindestens ein Substituent R mindestens ein sp³-hybridisiertes Kohlenstoffatom enthält.
Um eine deutlichere Abweichung von der Planarität zu erreichen, ist es bevorzugt, wenn dieses sp³-hybridisierte Kohlenstoffatom ein sekundäres, tertiäres oder quartäres Kohlenstoffatom ist, besonders bevorzugt ist ein tertiäres oder quartäres Kohlenstoffatom, ganz besonders bevorzugt ist ein quartäres Kohlenstoffatom.
Unter einem sekundären, tertiären oder quartären Kohlenstoffatom wird ein Kohlenstoffatom mit zwei, drei bzw. vier Substituenten ungleich Wasserstoff verstanden.

Besonders bevorzugt sind Verbindungen gemäß Formel (1), die in mindestens einem der Reste R ein 9,9'-Spirobifluorenderivat, ein 9,9-disubstituiertes Fluorenderivat, ein 6,6- und/oder 12,12-di- oder tetrasubstituiertes Indenofluorenderivat, ein Triptycenderivat (bevorzugt verknüpft über die Position 9 und/oder 10) oder ein Tetraarylmethanderivat enthalten. Dabei kann die Diazin- bzw. Triazin- bzw. Tetrazineinheit beispielsweise auch auch in 9-Position des Fluorens bzw. in 6- und/oder 12-Position des Indenofluorens gebunden sein.

Ganz besonders bevorzugt sind Verbindungen gemäß Formel (1), die in mindestens einem der Reste R ein 9,9'-Spirobifluorenderivat enthalten.

Die Glasübergangstemperatur der Verbindungen gemäß Formel (1) ist bevorzugt > 100 °C, besonders bevorzugt > 120 °C, ganz besonders bevorzugt > 140 °C. Es hat sich gezeigt, dass die Glasübergangstemperatur von Verbindungen, in denen mindestens einer der Reste R ein Spirobifluorenderivat enthält, meist in diesem Bereich liegen. Dies begründet die Bevorzugung dieser Materialien.

Es hat sich gezeigt, dass die besten Ergebnisse (in Bezug auf die Effizienz und die Lebensdauer) erzielt werden, wenn die Schichtdicke der Lochblockierschicht 1 bis 50 nm beträgt, bevorzugt 5 bis 30 nm.

Weiterhin hat sich gezeigt, dass besonders gute Ergebnisse, insbesondere in Bezug auf die Betriebsspannung und Leistungseffizienz, erzielt werden, wenn zwischen der Lochblockierschicht und der Kathode bzw. der Elektroneninjektionsschicht keine separate Elektronentransportschicht eingebracht wird. Bevorzugt ist also eine erfindungsgemäße Elektrolumineszenzvorrichtung, die keine Elektronentransportschicht enthält und in der die Lochblockierschicht direkt an die Elektroneninjektionsschicht bzw. die Kathode angrenzt. Dies ist ein überraschendes Ergebnis, da dieselbe Devicestruktur mit BCP als Lochblockiermaterial ohne ETL deutlich kürzere Lebensdauern liefert.

Die vorliegende Erfindung wird durch die folgenden Beispiele für Lochblockiermaterialien gemäß Formel (1) näher erläutert, ohne sie darauf einschränken zu wollen. Mögliche Substituenten an der Spirobifluoreneinheit bzw. den entsprechenden anderen Einheiten und auch mögliche weitere Substituenten am Triazin sind der Übersichtlichkeit halber nicht abgebildet. Der Fachmann kann aus der Beschreibung und den aufgeführten Beispielen ohne erfinderisches Zutun weitere erfindungsgemäße Elektrolumineszenzvorrichtungen mit ähnlichen Lochblockiermaterialien herstellen.

| | | |
|---|---|---|
| 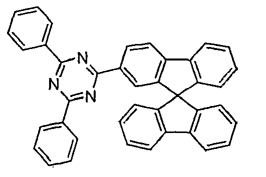 | 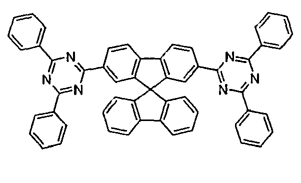 | 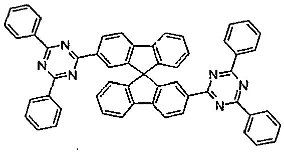 |
| Beispiel 1 | Beispiel 2 | Beispiel 3 |
| 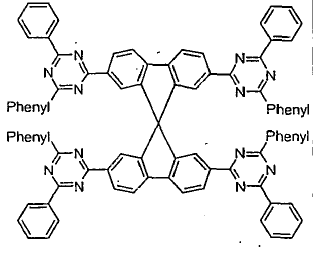 | 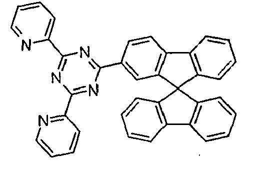 | 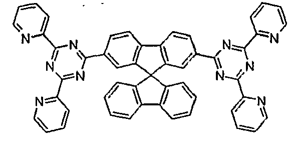 |
| Beispiel 4 | Beispiel 5 | Beispiel 6 |
| 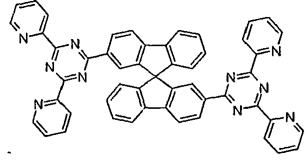 | 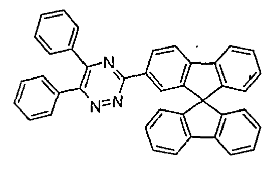 | 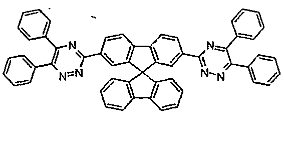 |
| Beispiel 7 | Beispiel 8 | Beispiel 9 |
| 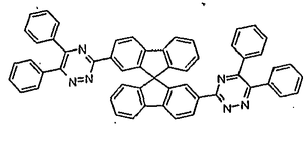 | 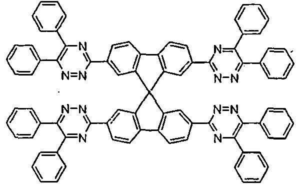 | 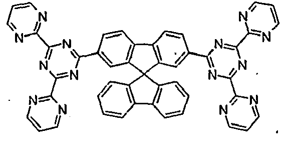 |
| Beispiel 10 | Beispiel 11 | Beispiel 12 |
| 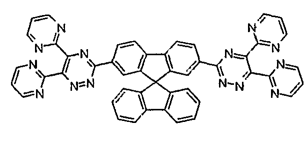 | 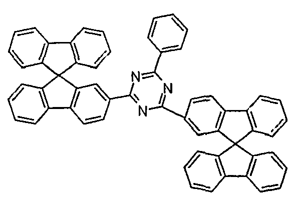 | 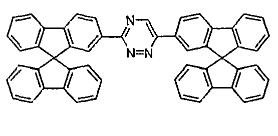 |
| Beispiel 13 | Beispiel 14 | Beispiel 15 |
| 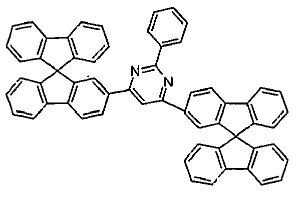 | 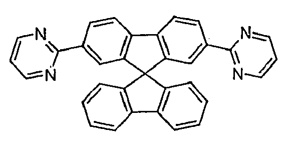 | 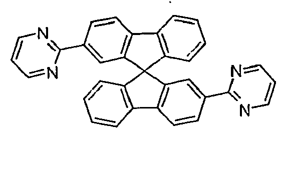 |
| Beispiel 16 | Beispiel 17 | Beispiel 18 |
| 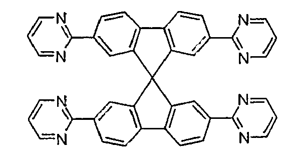 | 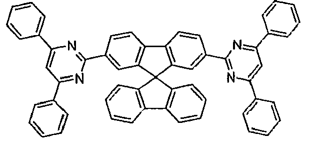 | 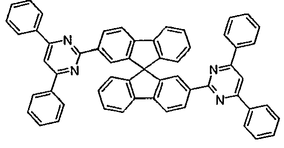 |
| Beispiel 19 | Beispiel 20 | Beispiel 21 |
| 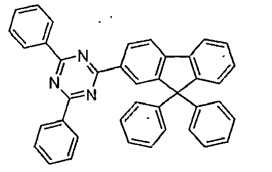 | 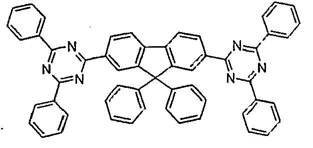 | 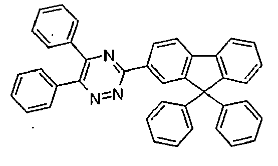 |
| Beispiel 22 | Beispiel 23 | Beispiel 24 |
| 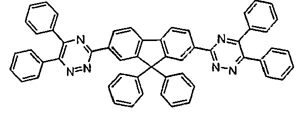 | 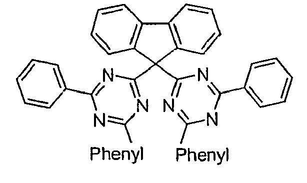 | 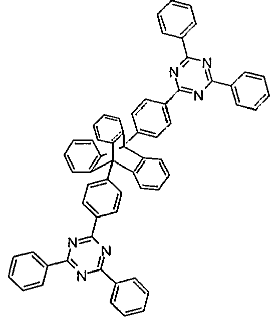 |
| Beispiel 25 | Beispiel 26 | Beispiel 27 |
| 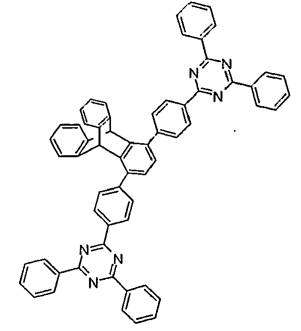 | 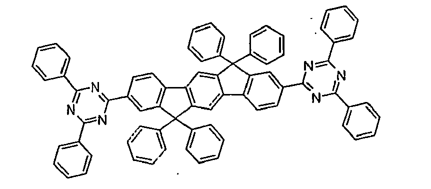 | |
| Beispiel 28 | Beispiel 29 | |
| 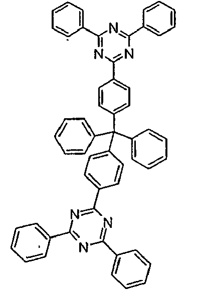 | 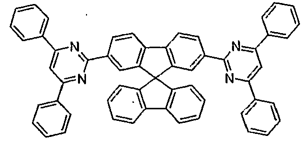 | 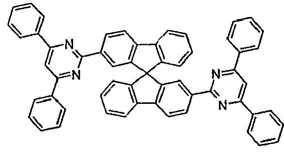 |
| Beispiel 30 | Beispiel 31 | Beispiel 32 |

Die Matrix für den phosphoreszierenden Emitter ist bevorzugt ausgewählt aus den Klassen der Carbazole, z. B. gemäß WO 00/057676, EP 1202358 und WO 02/074015, der Ketone und Imine, z. B. gemäß WO 04/093207, der Phosphinoxide, Phosphinsulfide, Phosphinselenide, der Phosphazene, der Sulfone, der Sulfoxide, z. B. gemäß DE 10330761.3, der Silane, der polypodalen Metallkomplexe, z. B. gemäß WO 04/081017, oder der Oligophenylene basierend auf Spirobifluorenen, z. B. gemäß EP 676461 und WO 99/40051; besonders bevorzugt sind Ketone, Phosphinoxide, Sulfoxide und Oligophenylene basierend auf Spirobifluorenen.

Der phosphoreszierende Emitter ist bevorzugt eine Verbindung, die mindestens ein Element der Ordnungszahl größer 36 und kleiner 84 aufweist.
Besonders bevorzugt enthält der phosphoreszierende Emitter mindestens ein Element der Ordnungszahl größer 56 und kleiner 80, ganz besonders bevorzugt Molybdän, Wolfram, Rhenium, Ruthenium, Osmium, Rhodium, Iridium, Palladium, Platin; Silber, Gold oder Europium, z. B. gemäß WO 98/01011, US 02/0034656, US 03/0022019, WO 00/70655, WO 01/41512, WO 02/02714, WO 02/15645, EP 1191613, EP 1191612, EP 1191614, WO 03/040257, WO 03/084972 und WO 04/026886.

Bevorzugt werden in der organischen Elektrolumineszenzvorrichtung eine oder mehrere Schichten mit einem Sublimationsverfahren beschichtet. Dabei werden die niedermolekularen Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Ebenfalls bevorzugt werden in der organischen Elektrolumineszenzvorrichtung eine oder mehrere Schichten mit dem OVPD-Verfahren (Organic Vapour Phase Deposition) oder mit Hilfe der Trägergassublimation beschichtet werden. Dabei werden die niedermolekularen Materialien bei einem Druck zwischen 10⁻⁵ mbar und 1 bar aufgebracht.

Ebenfalls bevorzugt werden in der organischen Elektrolumineszenzvorrichtung eine oder mehrere Schichten mit einem Druckverfahren, wie z. B. Flexodruck oder Offsetdruck, besonders bevorzugt aber LITI (Light Induced Thermal Imaging, Thermotransferdruck) oder Ink-Jet Druck (Tintenstrahl-Druck), beschichtet.

Die oben beschriebenen emittierenden Vorrichtungen weisen nun folgende überraschende Vorteile gegenüber dem Stand der Technik auf:
1. Die Effizienz entsprechender Vorrichtungen ist höher im Vergleich zu Systemen gemäß dem Stand der Technik, die BAlq als HBL enthalten.
2. Die Lebensdauer entsprechender Vorrichtungen ist höher im Vergleich zu Systemen, die BCP als HBL enthalten. Dadurch erhält man Vorrichtungen, deren Lebensdauer und Effizienz vergleichbar sind mit den besten Werten gemäß Stand der Technik und in denen nicht nur eine der beiden Eigenschaften gute Ergebnisse liefert, wie dies mit BAlq oder BCP der Fall ist.
3. Die Betriebsspannungen sind in erfindungsgemäßen Vorrichtungen niedriger als in Vorrichtungen gemäß Stand der Technik.
4. Der Schichtaufbau kann vereinfacht werden, weil keine separate Elektronentransportschicht verwendet werden muss. Dies ist ein überraschendes Ergebnis, da dieselbe Devicestruktur mit BCP statt Triazin ohne Elektronentransportschicht deutlich schlechtere Lebensdauern und Effizienzen liefert.
5. Wenn keine Elektronentransportschicht verwendet wird, ergibt sich ein weiterer Vorteil: Die Betriebsspannungen sind hier wesentlich geringer; dadurch erhöht sich die Leistungseffizienz erheblich. Dies ist ein überraschendes Ergebnis, da dieselbe Devicestruktur mit BAIq statt Triazin in kaum verringerter Betriebsspannung resultiert.
6. Der Produktionsaufwand wird ohne Verwendung einer Elektronentransportschicht ebenfalls geringer. Dies ist ein erheblicher technologischer Vorteil im Produktionsprozess, da bei der herkömmlichen Herstellungsweise für jede organische Schicht ein separates Segment der Aufdampfanlage benötigt wird.

Details zu den hier gemachten Angaben finden sich in den unten beschriebenen Beispielen.

Im vorliegenden Anmeldetext und in den folgenden Beispielen wird auf organische Leuchtdioden und die entsprechenden Displays abgezielt. Trotz dieser Beschränkung der Beschreibung ist es für den Fachmann ohne weiteres erfinderisches Zutun möglich, das entsprechende erfindungsgemäße Design auch für andere, verwandte Vorrichtungen, z. B. für organische Solarzellen (O-SCs), organische Transistoren, organische integrierte Schaltungen oder auch organische Laserdioden (O-Laser), um nur einige weitere Anwendungen zu nennen, zu verwenden. Diese sind also auch Gegenstand der vorliegenden Anmeldung.

Spirobifluorene, die mit Triazinen substituiert sind, sind neu und somit ebenfalls Gegenstand der vorliegenden Erfindung.

Gegenstand der Erfindung sind also weiterhin Verbindungen gemäß Formel (2), enthaltend mindestens eine Spirobifluoreneinheit, **dadurch gekennzeichnet, dass** mindestens eine Triazineinheit an das Spirobifluoren gebunden ist, 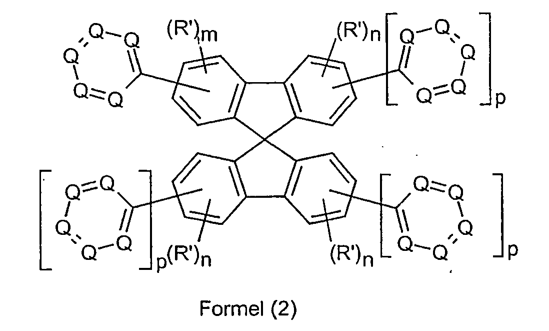 wobei R und R¹ dieselbe Bedeutung haben, wie oben unter Formel (1) definiert und die weiteren Symbole und Indizes die folgende Bedeutung haben:
- Q: ist bei jedem Auftreten gleich oder verschieden N oder CR mit der Maßgabe, dass drei Q für Stickstoff und zwei Q für CR stehen;
- R': ist bei jedem Auftreten gleich oder verschieden R oder F, Cl, Br, I, B(R¹)₂ oder B(OR¹)₂;
- m: ist bei jedem Auftreten gleich oder verschieden 0, 1, 2 oder 3;
- n: ist bei jedem Auftreten gleich oder verschieden 0, 1, 2, 3 oder 4 mit der Maßgabe, dass n nicht 4 sein darf, wenn p = 1 ist;
- p: ist bei jedem Auftreten gleich oder verschieden 0 oder 1;
ausgenommen ist dabei die folgende Verbindung

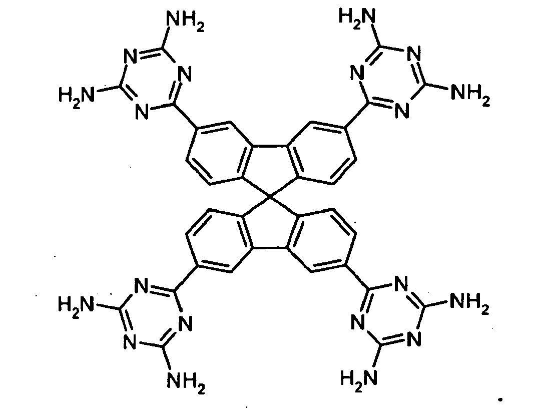

Bei der Triazineinheit kann es sich erfindungsgemäß um 1,2,3-, 1,2,4- oder 1,3,5-Triazin handeln. Dabei können auch verschiedene Triazinderivate in einer Verbindung enthalten sein. Bevorzugt handelt es sich bei allen in einer Verbindung enthaltenen Triazinen um das gleiche Triazinderivat. Besonders bevorzugt handelt es sich um 1,3,5-Triazin oder um 1,2,4-Triazin.

Die Verknüpfung der Triazineinheit mit dem Spirobifluoren erfolgt bevorzugt in Position 2 (bzw. 2', 7 oder 7'), also in *para*-Position zur Phenyl-Phenyl-Verknüpfung des Spirobifluorens.

In bevorzugten Strukturen gemäß Formel (2) gilt für die Symbole und Indizes Folgendes:
- R: ist bei jedem Auftreten gleich oder verschieden eine geradkettige, verzweigte oder cyclische Alkylgruppe mit 1 bis 10 C-Atomen, wobei eine oder mehrere nicht benachbarte CH₂-Gruppen durch -R¹C=CR¹-, -C≡C-, Si(R¹)₂, Ge(R¹)₂, Sn(R¹)₂, -O-, -S- oder -NR¹- ersetzt sein können, oder ein aromatisches oder heteroaromatisches Ringsystem mit 1 bis 30 aromatischen C-Atomen, das durch einen oder mehrere nicht aromatische Reste R substituiert sein kann, wobei mehrere Substituenten R, sowohl am selben Ring als auch an unterschiedlichen Ringen zusammen wiederum ein weiteres mono- oder polycyclisches, aliphatisches oder aromatisches Ringsystem aufspannen können;
- m: ist bei jedem Auftreten gleich oder verschieden 0 oder 1;
- n: ist bei jedem Auftreten gleich oder verschieden 0 oder 1;
die weiteren Symbole und Indizes sind wie oben unter Formel (1) und (2) definiert.

In besonders bevorzugten Strukturen gemäß Formel (2) gilt für die Symbole und Indizes Folgendes:
- R: ist bei jedem Auftreten gleich oder verschieden ein aromatisches oder heteroaromatisches Ringsystem mit 1 bis 10 aromatischen C-Atomen, die durch einen oder mehrere nicht aromatische Reste R, wie oben definiert, substituiert sein kann, wobei mehrere Substituenten R, sowohl am selben Ring als auch an unterschiedlichen Ringen zusammen wiederum ein weiteres mono- oder polycyclisches, aliphatisches oder aromatisches Ringsystem aufspannen können;
- R': ist bei jedem Auftreten gleich oder verschieden R, eine geradkettige, verzweigte oder cyclische Alkylgruppe mit 1 bis 10 C-Atomen, wobei eine oder mehrere nicht benachbarte CH₂-Gruppen durch -R¹C=CR¹-, -C≡C-, Si(R¹)₂, Ge(R¹)₂, Sn(R¹)₂, -O-, -S- oder -NR¹- ersetzt sein können, oder Br, I oder B(OR¹)₂;
- m: ist gleich 0;
- n: ist bei jedem Auftreten gleich oder verschieden 0 oder 1;
die weiteren Symbole und Indizes sind wie oben unter Formel (1) und (2) definiert.

Bevorzugt sind weiterhin Verbindungen gemäß Formel (2), in denen zwei TriazinEinheiten vorhanden sind, die beide an dieselbe Fluoren-Untereinheit des Spirobifluorens gebunden sind, bevorzugt in Position 2 und 7.

Die oben beschriebenen erfindungsgemäßen Verbindungen gemäß Formel (2) können beispielsweise auch als Comonomere zur Erzeugung entsprechender Polymere oder auch als Kern von Dendrimeren Verwendung finden. Dafür eignen sich besonders Verbindungen gemäß Formel (2), die entsprechende Funktionalitäten enthalten, die sich zur Folgereaktion eignen, wie beispielsweise Halogene, insbesondere Brom und Iod, oder Boronsäuren oder entsprechende Derivate. So können diese Verbindungen u. a. in lösliche Polyfluorene (z. B. gemäß EP 842208 oder WO 00/22026), Poly-spirobifluorene (z. B. gemäß EP 707020 oder EP-A-894107), Poly-indenofluorene (z. B. gemäß WO 04/041901 oder EP 03014042.0) oder Poly-dihydrophenanthrene (z. B. gemäß DE 10337346.2) einpolymerisiert werden. Diese Polymere oder Dendrimere können als Lochblockiermaterial in organischen Elektrolumineszenzvorrichtungen verwendet werden.

Weiterhin können die Materialien gemäß Formel (2) auch durch die beispielsweise o. g. Reaktionstypen weiter funktionalisiert werden, und so zu erweiterten Lochblockiermatialien gemäß Formel (2) umgesetzt werden. Hier ist als Beispiel die Funktionalisierung mit Arylboronsäuren gem. SUZUKI oder mit Aminen gem. HARTWIG-BUCHWALD zu nennen.

Weiterhin Gegenstand der vorliegenden Erfindung ist die Verwendung von Verbindungen gemäß Formel (2) oder auch Polymeren oder Dendrimeren, die diese Verbindungen enthalten, in elektronischen Vorrichtungen. Ebenso Gegenstand der Erfindung sind elektronische Vorrichtungen, wie zum Beispiel organische Leuchtdioden, organische Solarzellen, organische Transistoren, organische integrierte Schaltungen oder organische Laserdioden, die mindestens eine Verbindung gemäß Formel (2) oder ein entsprechendes Polymer oder Dendrimer enthalten.

Die Herstellung der OLEDs erfolgt nach einem allgemeinen Verfahren, das im Einzelfall auf die jeweiligen Gegebenheiten (z. B. Schichtdickenvariation zur Optimierung der Effizienz bzw. der Farbe) angepasst wurde. Für die Herstellung der erfindungsgemäßen Vorrichtungen wurde als Lochblockierschicht eine Verbindung gemäß Formel (1) verwendet und optional die Elektronentransportschicht weggelassen. Erfindungsgemäße Elektrolumineszenzvorrichtungen können wie beispielsweise in DE10330761.3 beschrieben dargestellt werden.

### Beispiele:

Die nachfolgenden Synthesen wurden, sofern nicht anders angegeben, unter einer Schutzgasatmosphäre in getrockneten Lösungsmitteln durchgeführt. Die Edukte wurden von ALDRICH (Kaliumfluorid (sprühgetrocknet), Tri-*tert*-butylphosphin, Palladium(II)acetat) bezogen. 3-Chlor-5,6-diphenyl-1,2,4-triazin wurde von SYNCHEM OHG bezogen. 2',7'-Di-*tert*-butyl-spiro-9,9'-bifluoren-2,7-bisboronsäureglycolester wurde nach WO 02/077060 und 2-Chlor-4,6-diphenyl-1,3,5-triazin wurde nach US 5438138 dargestellt. Spiro-9,9'-bifluoren-2,7-bis(boronsäureglycolester) wurde analog zu WO 02/077060 dargestellt.

### Beispiel 1: Synthese von 2,7-Bis(4,6-diphenyl-1,3,5-triazin-2-yl)-2',7'-di-tert-butyl-spiro-9,9'-bifluoren (TRI1)

28.4 g (50.0 mmol) 2',7'-Di-*tert*-butyl-spiro-9,9'-bifluoren-2,7-bisboronsäureglycolester, 29.5 g (110.0 mmol) 2-Chlor-4,6-diphenyl-1,3,5-triazin und 44.6 g (210.0 mmol) Trikaliumphosphat wurden in 500 mL Toulol, 500 mL Dioxan und 500 mL Wasser suspendiert. Zu dieser Suspension wurden 913 mg (3.0 mmol) Tri-o-tolylphosphin und dann 112 mg (0.5 mmol) Palladium(II)acetat gegeben, und die Reaktionsmischung wurde 16 h unter Rückfluss erhitzt. Nach Erkalten wurde die organische Phase abgetrennt, über Kieselgel filtriert, dreimal mit 200 mL Wasser gewaschen und anschließend zur Trockene eingeengt. Der Rückstand wurde aus Toluol und aus Dichlormethan / *iso*-Propanol umkristallisiert und abschließend im Hochvakuum (p = 5 x 10⁻⁵ mbar, T = 385 °C) sublimiert. Die Ausbeute betrug 39.9 g (44.8 mmol), entsprechend 89.5 % der Theorie.
¹H-NMR (CDCl₃): [ppm] = 8.89 (m, 2H), 8.65 (m, 8H), 8.14 (m, 2H), 8.06 (m, 2H), 7.86 (m, 2H), 7.61-7.50 (m, 12H), 7.47 (m, 2H), 6.79 (m, 2H), 1.16 (s, 18 H).

### Beispiel 2: Synthese von 2,7-Bis(4,6-diphenyl-1,3,5-triazin-2-yl)spiro-9,9'-bifluoren (TRI2)

Durchführung analog Beispiel 1, wobei 2',7'-Di-*tert*-butyl-spiro-9,9'-bifluoren-2,7-bis(boronsäureglycolester) durch 22.8 g (50 mmol) Spiro-9,9'-bifluoren-2,7-bis(boronsäureglycolester) ersetzt wurde. Die Ausbeute betrug 32.3 g (41.5 mmol), entsprechend 82.9 % der Theorie.
¹H-NMR (CDCl₃): [ppm] = 8.90 (m, 2H), 8.64 (m, 8H), 8.14 (m, 2H), 8.09 (m, 2H), 8.01 (m, 2H), 7.61-7.49 (m, 12H), 7.45 (m, 2H), 7.15 (m, 2), 6.86 (m, 2H).

### Beispiel 3: Synthese von 2,7-Bis(5,6-diphenyl-1,2,4-triazin-3-yl)-2',7'-di-tert-butyl-spiro-9,9'-bifluoren (TRI3)

Durchführung analog Beispiel 1, wobei das 2-Chlor-4,6-diphenyl-1,3,5-triazin durch 3-Chlor-5,6-diphenyl-1,2,4-triazin ersetzt wurde. Die Ausbeute betrug 41.0 g (46.0 mmol), entsprechend 92.0 % der Theorie.
¹H-NMR (CDCl₃): [ppm] = 8.74 (m, 2H), 8.12 (m, 4H), 7.75 (m, 2H), 7.59 (m, 4H), 7.53 (m, 4H), 7.45-7.30 (m, 14H), 6.76 (m, 2H) 1.14 (s, 18 H).

### Beispiel 4: Deviceaufbau

Die folgenden Beispiele zeigen die Ergebnisse verschiedener OLEDs, sowohl mit Lochblockiermaterialien gemäß Formel (1) wie auch mit BCP und BAlq als Vergleichsmaterialien. Der grundlegende Aufbau, die verwendeten Materialien und Schichtdicken (außer der HBLs) waren zur besseren Vergleichbarkeit identisch. Gemäß dem o. g. allgemeinen Verfahren wurden phosphoreszierende OLEDs mit folgendem Aufbau erzeugt:
- PEDOT (HIL): 60 nm (aus Wasser aufgeschleudert; bezogen als Baytron P von H. C. Stark; Poly-(3,4-ethylendioxy-2,5-thiophen))
- NaphDATA (HTL): 20 nm (aufgedampft; bezogen von SynTec; 4,4',4"-Tris(N-1-naphthyl-N-phenylamino)-triphenylamin)
- S-TAD (HTL): 20 nm (aufgedampft; hergestellt nach WO 99/12888; 2,2',7,7'-Tetrakis(diphenylamino)-spirobifluoren)
- (EML): 30 nm (aufgedampft); 10 % IrPPy in Bis(9,9'-spirobifluoren-2-yl)keton als Matrixmaterial
- (HBL): Materialien und Schichtdicken siehe Beispiele in Tabelle 1
- AIQ₃ (ETL): nicht in allen Devices vorhanden (siehe Tabelle 1); wenn vorhanden: aufgedampft (bezogen von SynTec; Tris(8-hydroxychinolato)aluminium(III))
- Ba-Al (Kathode): 3 nm Ba, darauf 150 nm Al.

Diese noch nicht optimierten OLEDs wurden standardmäßig charakterisiert; hierfür wurden die Elektrolumineszenzspektren, die Effizienz (gemessen in cd/A), die Leistungseffizienz (gemessen in Im/W) in Abhängigkeit der Helligkeit und die Lebensdauer bestimmt. Als Lebensdauer wird die Zeit definiert, nach der die Anfangshelligkeit der OLED bei einer konstanten Stromdichte von 10 mA/cm² auf die Hälfte abgesunken ist.

In Tabelle 1 sind die Ergebnisse der erfindungsgemäßen OLEDs und einiger Vergleichsbeispiele (mit BCP und BAIq) zusammengefasst (Beispiele 5 bis 8). In der Tabelle ist lediglich die Lochblockierschicht (Zusammensetzung und Schichtdicke) aufgeführt. Die anderen Schichten entsprechen dem oben genannten Aufbau. In Beispiel 5 wird **TRI1** zusammen mit einer ETL verwendet, in Beispiel 6 ohne ETL. In Beispiel 7 wird **TRI2** zusammen mit einer ETL verwendet, in Beispiel 8 ohne ETL.

Die oben bzw. in der Tabelle 1 verwendeten Abkürzungen entsprechen den folgenden Verbindungen:

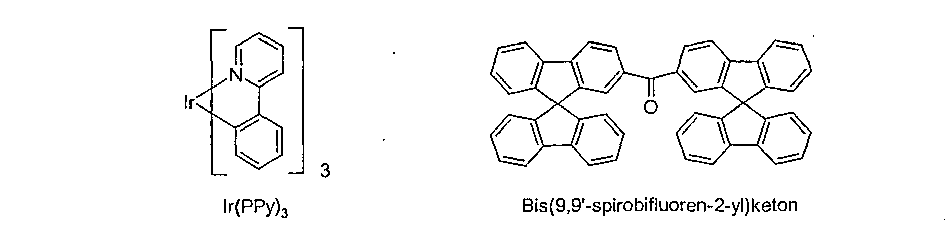

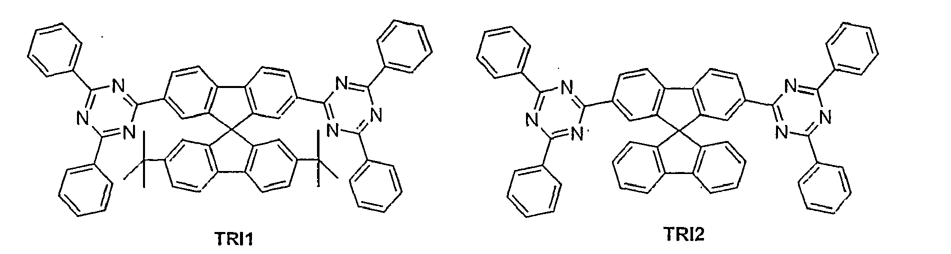

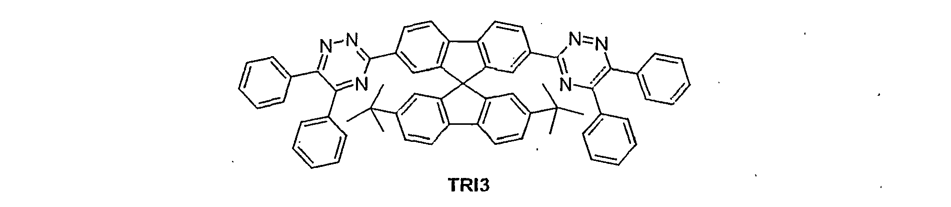

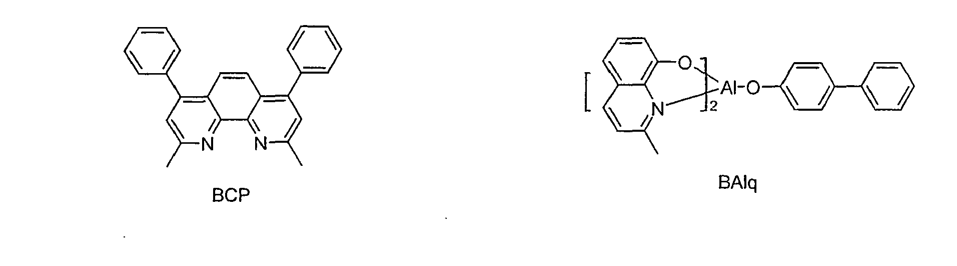

**Tabelle 1:**

| Beispiel | HBL | ETL | Max. Effizienz (cd/A) | Spannung (V) bei 100 cd/m² | Leistungseff. (Im/W) bei max. Effizienz | CIE (x, y) | Lebensdauer (h) bei 10 mA/cm² |
|---|---|---|---|---|---|---|---|
| Beispiel 5a | **TRI1** (10 nm) | AlQ₃ (20 nm) | 33.5 | 4.3 | 22.1 | 0.39/0.57 | 780 |
| Beispiel 5b (Vergleich) | BAIq (10 nm) | AlQ₃ (20 nm) | 25.2 | 5.7 | 14.8 | 0.39/0.57 | 510 |
| Beispiel 5c (Vergleich) | BCP (10 nm) | AlQ₃ (20 nm) | 32.6 | 4.8 | 18.2 | 0.39/0.57 | 360 |
| Beispiel 6a | **TRI1** (20 nm) | ― | 34.2 | 2.7 | 41.4 | 0.38/0.58 | 310 |
| Beispiel 6b (Vergleich) | BAIq (20 nm) | ― | 24.8 | 5.2 | 14.7 | 0.39/0.58 | 240 |
| Beispiel6c (Vergleich) | BCP (20 nm) | ― | 16.7 | 4.8 | 8.7 | 0.32/0.62 | 80 |
| Beispiel 7a | **TRI2** (10 nm) | AlQ₃ (20 nm) | 30.9 | 4.0 | 19.2 | 0.39/0.58 | 770 |
| Beispiel 7b (Vergleich) | s. Beispiel 5b und 5c | | | | | | |
| Beispiel 8a | **TRI2** (20 nm) | ― | 32.9 | 2.9 | 33.8 | 0.38/0.58 | 320 |
| Beispiel 8b (Vergleich) | s. Beispiel 6b und 6c | | | | | | |

Zusammenfassend kann gesagt werden, dass phosphoreszierende OLEDs, die Lochblockiermaterialien gemäß Formel (1) bzw. gemäß Formel (2) enthalten, hohe Effizienzen bei gleichzeitig langen Lebensdauern und niedrigen Betriebsspannungen aufweisen, wie man leicht den Beispielen aus Tabelle 1 entnehmen kann. Insbesondere ohne Verwendung einer Elektronentransportschicht werden sehr niedrige Betriebsspannungen und sehr hohe Leistungseffizienzen erhalten.

## Patentansprüche

1. Organische Elektrolumineszenzvorrichtung, enthaltend eine Anode, eine Kathode und eine Emissionsschicht, bestehend aus mindestens einem Matrixmaterial, welches mit mindestens einem phosphoreszierenden Emitter dotiert ist, **dadurch gekennzeichnet, dass** zwischen die Emissionsschicht und die Kathode eine Lochblockierschicht eingebracht ist, die eine Verbindung gemäß Formel (1) enthält, 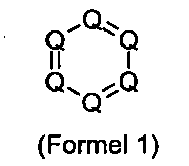 wobei für die verwendeten Symbole und Indizes gilt:
Q ist bei jedem Auftreten gleich oder verschieden N oder CR mit der Maßgabe, dass mindestens zwei und maximal vier Q für Stickstoff stehen;
R ist bei jedem Auftreten gleich oder verschieden H, NO₂, CN, N(R¹)₂, eine geradkettige, verzweigte oder cyclische Alkyl- oder Alkoxygruppe mit 1 bis 40 C-Atomen, wobei eine oder mehrere nicht benachbarte CH₂-Gruppen durch -R¹C=CR¹-, -C≡C-, Si(R¹)₂, Ge(R¹)₂, Sn(R¹)₂, -O-, -S- oder -NR¹- ersetzt sein können und wobei ein oder mehrere H-Atome durch F oder eine aromatische Gruppe R¹ ersetzt sein können, oder
ein aromatisches bzw. heteroaromatisches Ringsystem oder eine Aryloxy- oder Heteroaryloxygruppe jeweils mit 1 bis 40 aromatischen C-Atomen, wobei ein oder mehrere H-Atome durch F, Cl, Br oder I ersetzt sein können oder die durch einen oder mehrere nicht-aromatische Reste R substituiert sein kann; dabei können mehrere Substituenten R ein weiteres mono- oder polycyclisches, aliphatisches oder aromatisches Ringsystem aufspannen; oder
ein über eine bivalente Gruppe -Z- gebundenes aromatisches bzw. heteroaromatisches Ringsystem oder eine Aryloxy- oder Heteroaryloxygruppe jeweils mit 1 bis 40 aromatischen C-Atomen, wobei ein oder mehrere H-Atome durch F, Cl, Br oder I ersetzt sein können oder die durch einen oder mehrere nicht aromatische Reste R substituiert sein kann; dabei können mehrere Substituenten R ein weiteres mono- oder polycyclisches, aliphatisches oder aromatisches Ringsystem aufspannen;
R¹ ist bei jedem Auftreten gleich oder verschieden H oder ein aliphatischer, aromatischer oder heteroaromatischer Kohlenwasserstoffrest mit 1 bis 20 C-Atomen, wobei mehrere Substituenten R¹ bzw. R¹ mit R auch ein weiteres mono- oder polycyclisches, aliphatisches oder aromatisches Ringsystem aufspannen können;
Z ist bei jedem Auftreten gleich oder verschieden ein geradkettiger, verzweigter oder cyclischer, bevorzugt konjugierter Rest mit 1 bis 40 C-Atomen, der bevorzugt in Konjugation mit den beiden anderen Substituenten steht, wobei bevorzugt die Anzahl der Atome von Z, die die Gruppe gemäß Formel (1) und den aromatischen Rest verknüpfen, eine gerade Zahl beträgt, wobei ein oder mehrere nicht benachbarte C-Atome durch -O-, -S- oder -NR¹- ersetzt sein können oder ein oder mehrere C-Atome durch einen Rest R¹ oder Halogen substituiert sein können; mit der Maßgabe, dass R kein substituiertes oder unsubstituiertes Phenylpyridin enthält;
**dadurch gekennzeichnet, dass** in mindestens einem der Reste R ein 9,9'-Spirobifluorenderivat, ein 9,9-disubstituiertes Fluorenderivat, ein 6,6- und/oder 12,12-di- oder tetrasubstituiertes Indenofluorenderivat, ein Tetraarylmethanderivat oder ein Triptycenderivat enthalten ist.

2. Organische Elektrolumineszenzvorrichtung gemäß Anspruch 1, **dadurch gekennzeichnet, dass** in Verbindungen gemäß Formel (1) in mindestens einem der Reste R ein 9,9'-Spirobifluorenderivat enthalten ist.

3. Organische Elektrolumineszenzvorrichtung gemäß Anspruch 1 oder 2, **dadurch gekennzeichnet, dass** eine Lochinjektionsschicht und/oder eine Lochtransportschicht und/oder eine Elektroneninjektionsschicht und/oder eine Elektronentransportschicht enthalten ist.

4. Organische Elektrolumineszenzvorrichtung gemäß einem oder mehreren der Ansprüche 1 bis 3, **dadurch gekennzeichnet, dass** die Lochblockierschicht mindestens 50 % Verbindungen gemäß Formel (1) enthält.

5. Organische Elektrolumineszenzvorrichtung gemäß Anspruch 4, **dadurch gekennzeichnet, dass** die Lochblockierschicht nur aus Verbindungen gemäß Formel (1) besteht.

6. Organische Elektrolumineszenzvorrichtung gemäß einem oder mehreren der Ansprüche 1 bis 5, **dadurch gekennzeichnet, dass** die Strukturen gemäß Formel (1) ausgesucht sind aus den Gruppen der Pyridazine, Pyrimidine, Pyrazine, 1,2,3-, 1,2,4- oder 1,3,5-Triazine.

7. Organische Elektrolumineszenzvorrichtung gemäß Anspruch 6, **dadurch gekennzeichnet, dass** die Strukturen gemäß Formel (1) ausgesucht sind aus den Gruppen der 1,2,4-Triazine oder der 1,3,5-Triazine.

8. Organische Elektrolumineszenzvorrichtung gemäß einem oder mehreren der Ansprüche 1 bis 7, **dadurch gekennzeichnet, dass** das Lochblockiermaterial mehr als eine Einheit gemäß Formel (1) enthält.

9. Organische Elektrolumineszenzvorrichtung gemäß einem oder mehreren der Ansprüche 1 bis 8, **dadurch gekennzeichnet, dass** die Glasübergangstemperatur der Verbindungen gemäß Formel (1) > 100 °C ist.

10. Organische Elektrolumineszenzvorrichtung gemäß einem oder mehreren der Ansprüche 1 bis 9, **dadurch gekennzeichnet, dass** die Schichtdicke der Lochblockierschicht 1 bis 50 nm beträgt.

11. Organische Elektrolumineszenzvorrichtung gemäß einem oder mehreren der Ansprüche 1 bis 10, **dadurch gekennzeichnet, dass** die Matrix für den phosphoreszierenden Emitter ausgewählt ist aus den Klassen der Carbazole, Ketone, Imine, Phosphinoxide, Phosphinsulfide, Phosphinselenide, Phosphazene, Sulfone, Sulfoxide, Silane, polypodalen Metallkomplexe oder Oligophenylene basierend auf Spirobifluorenen.

12. Organische Elektrolumineszenzvorrichtung gemäß einem oder mehreren der Ansprüche 1 bis 11, **dadurch gekennzeichnet, dass** der phosphoreszierende Emitter eine Verbindung ist, die mindestens ein Element der Ordnungszahl größer 36 und kleiner 84 aufweist.

13. Organische Elektrolumineszenzvorrichtung gemäß Anspruch 12, **dadurch gekennzeichnet, dass** der phosphoreszierende Emitter mindestens ein Element enthält, ausgesucht aus den Elementen Molybdän, Wolfram, Rhenium, Ruthenium, Osmium, Rhodium, Iridium, Palladium, Platin, Silber, Gold oder Europium.

14. Organische Elektrolumineszenzvorrichtung gemäß einem oder mehreren der Ansprüche 1 bis 13, **dadurch gekennzeichnet, dass** eine oder mehrere Schichten mit einem Sublimationsverfahren beschichtet werden.

15. Organische Elektrolumineszenzvorrichtung gemäß einem oder mehreren der Ansprüche 1 bis 13, **dadurch gekennzeichnet, dass** eine oder mehrere Schichten mit dem OVPD-Verfahren (Organic Vapour Phase Deposition) oder mit Hilfe der Trägergassublimation beschichtet werden.

16. Organische Elektrolumineszenzvorrichtung gemäß einem oder mehreren der Ansprüche 1 bis 13, **dadurch gekennzeichnet, dass** eine oder mehrere Schichten mit einem Druckverfahren beschichtet werden.

17. Verwendung des Designs der elektronischen Vorrichtungen gemäß einem oder mehreren der Ansprüche 1 bis 13 für organische Transistoren, organische integrierte Schaltungen, organische Solarzellen, organische Laserdioden oder Photorezeptoren.

18. Verbindungen gemäß Formel (2), enthaltend mindestens eine 9,9'-Spirobifluoreneinheit, **dadurch gekennzeichnet, dass** mindestens eine Triazineinheit an das 9,9'-Spirobifluoren gebunden ist, 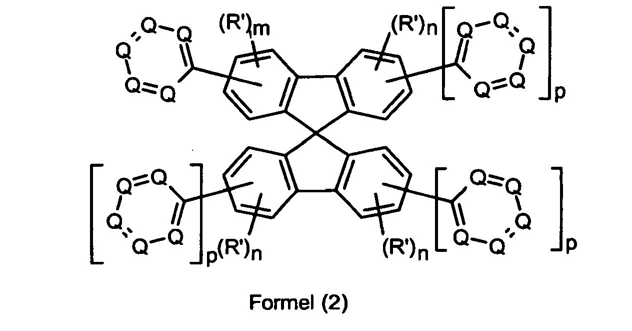 wobei R und R¹ dieselbe Bedeutung haben, wie in Anspruch 1 definiert und die weiteren Symbole und Indizes die folgende Bedeutung haben:
Q ist bei jedem Auftreten gleich oder verschieden N oder CR mit der Maßgabe, dass drei Q für Stickstoff und zwei Q für CR stehen;
R' ist bei jedem Auftreten gleich oder verschieden R oder F, Cl, Br, I, B(R¹)₂ oder B(OR¹)₂;
m ist bei jedem Auftreten gleich oder verschieden 0, 1, 2 oder 3;
n ist bei jedem Auftreten gleich oder verschieden 0, 1, 2, 3 oder 4 mit der Maßgabe, dass n nicht 4 sein darf, wenn p = 1 ist;
p ist bei jedem Auftreten gleich oder verschieden 0 oder 1;
ausgenommen ist dabei die folgende Verbindung

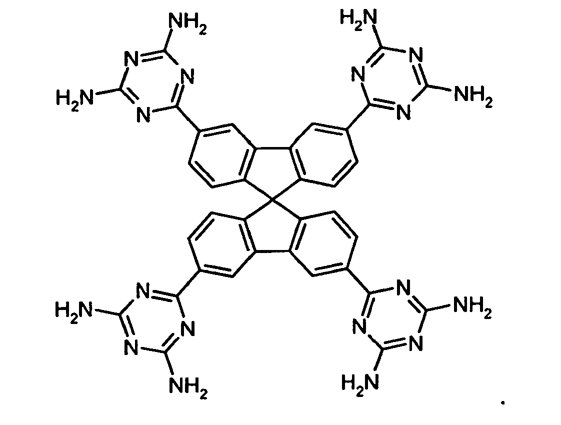

19. Verbindungen gemäß Anspruch 18, **dadurch gekennzeichnet, dass** es sich um 1,2,4-Triazin oder um 1,3,5-Triazin handelt.

20. Verbindungen gemäß Anspruch 18 oder 19, **dadurch gekennzeichnet, dass** für die Symbole und Indizes gilt:
R ist bei jedem Auftreten gleich oder verschieden ein aromatisches oder heteroaromatisches Ringsystem mit 1 bis 10 aromatischen C-Atomen, das durch einen oder mehrere nicht aromatische Reste R substituiert sein kann, wobei mehrere Substituenten R, sowohl am selben Ring als auch an unterschiedlichen Ringen zusammen wiederum ein weiteres mono- oder polycyclisches, aliphatisches oder aromatisches Ringsystem aufspannen können;
R' ist bei jedem Auftreten gleich oder verschieden R, eine geradkettige, verzweigte oder cyclische Alkylgruppe mit 1 bis 10 C-Atomen, wobei eine oder mehrere nicht benachbarte CH₂-Gruppen durch -R¹C=CR¹-, -C≡C-, Si(R¹)₂, Ge(R¹)₂, Sn(R¹)₂, -O-, -S- oder -NR¹- ersetzt sein können, oder Br, I oder B(OR¹)₂;
m ist bei jedem Auftreten gleich 0;
n ist bei jedem Auftreten gleich oder verschieden 0 oder 1;
die weiteren Symbole und Indizes sind wie oben unter Formel (1) und (2) definiert.

21. Verbindungen gemäß einem oder mehreren der Ansprüche 18 bis 20, **dadurch gekennzeichnet, dass** zwei Triazineinheiten vorhanden sind, die beide an dieselbe Fluoren-Untereinheit des Spirobifluorens gebunden sind.

22. Polymere oder Dendrimere enthaltend eine oder mehrere Verbindungen gemäß einem oder mehreren der Ansprüche 18 bis 21.

23. Verwendung von Verbindungen, Polymeren oder Dendrimeren gemäß einem oder mehreren der Ansprüche 18 bis 22 in elektronischen Vorrichtungen.

24. Elektronische Vorrichtung, enthaltend mindestens eine Verbindung, Polymer oder Dendrimer gemäß einem oder mehreren der Ansprüche 18 bis 23.

25. Elektronische Vorrichtung gemäß Anspruch 24, **dadurch gekennzeichnet, dass** es sich um eine organische Leuchtdiode, eine organische Solarzelle, einen organischen Transistor, eine organische integrierte Schaltung, eine organische Laserdiode oder einen organischen Photorezeptor handelt.

## Claims

1. Organic electroluminescent device comprising an anode, a cathode and an emission layer, consisting of at least one matrix material which is doped with at least one phosphorescent emitter, **characterised in that** a hole-blocking layer which comprises a compound of the formula (1) 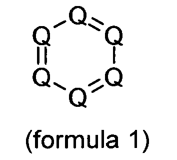 where the following applies to the symbols and indices used:
Q is on each occurrence, identically or differently, N or CR, with the proviso that at least two and a maximum of four Q stand for nitrogen;
R is on each occurrence, identically or differently, H, NO₂, CN, N(R¹)₂, a straight-chain, branched or cyclic alkyl or alkoxy group having 1 to 40 C atoms, in which one or more non-adjacent CH₂ groups may be replaced by -R¹C=CR¹-, -C≡C-, Si(R¹)₂, Ge(R¹)₂, Sn(R¹)₂, -O-, -S- or -NR¹- and in which one or more H atoms may be replaced by F or an aromatic group R¹, or an aromatic or heteroaromatic ring system or an aryloxy or heteroaryloxy group, each having 1 to 40 aromatic C atoms, in which one or more H atoms may be replaced by F, Cl, Br or I or which may be substituted by one or more non-aromatic radicals R; a plurality of substituents R here may form a further mono- or polycyclic, aliphatic or aromatic ring system;
or an aromatic or heteroaromatic ring system, which is bonded via a divalent group -Z-, or an aryloxy or heteroaryloxy group, each having 1 to 40 aromatic C atoms, in which one or more H atoms may be replaced by F, Cl, Br or I or which may be substituted by one or more non-aromatic radicals R; a plurality of substituents R here may form a further mono- or polycyclic, aliphatic or aromatic ring system;
R¹ is on each occurrence, identically or differently, H or an aliphatic, aromatic or heteroaromatic hydrocarbon radical having 1 to 20 C atoms, in which a plurality of substituents R¹ or R¹ with R may also form a further mono- or polycyclic, aliphatic or aromatic ring system;
Z is on each occurrence, identically or differently, a straight-chain, branched or cyclic, preferably conjugated radical having 1 to 40 C atoms, which is preferably in conjugation with the two other substituents, where the number of atoms in Z which link the group of the formula (1) and the aromatic radical is preferably an even number, where one or more non-adjacent C atoms may be replaced by -O-, -S- or -NR¹- or one or more C atoms may be substituted by a radical R¹ or halogen;
with the proviso that R does not contain substituted or unsubstituted phenyl-pyridine;
**characterised in that** a 9,9'-spirobifluorene derivative, a 9,9-disubstituted fluorene derivative, a 6,6- and/or 12,12-di- or tetrasubstituted indenofluorene derivative, a tetraarylmethane derivative or a triptycene derivative is present in at least one of the radicals R,
is incorporated between the emission layer and the cathode.

2. Organic electroluminescent device according to Claim 1, **characterised in that** a 9,9'-spirobifluorene derivative is present in at least one of the radicals R in compounds of the formula (1).

3. Organic electroluminescent device according to Claim 1 or 2, **characterised in that** a hole-injection layer and/or a hole-transport layer and/or an electron-injection layer and/or an electron-transport layer is present.

4. Organic electroluminescent device according to one or more of Claims 1 to 3, **characterised in that** the hole-blocking layer comprises at least 50% of compounds of the formula (1).

5. Organic electroluminescent device according to Claim 4, **characterised in that** the hole-blocking layer consists only of compounds of the formula (1).

6. Organic electroluminescent device according to one or more of Claims 1 to 5, **characterised in that** the structures of the formula (1) are selected from the groups of the pyridazines, pyrimidines, pyrazines, 1,2,3-, 1,2,4- or 1,3,5-triazines.

7. Organic electroluminescent device according to Claim 6, **characterised in that** the structures of the formula (1) are selected from the groups of the 1,2,4-triazines or 1,3,5-triazines.

8. Organic electroluminescent device according to one or more of Claims 1 to 7, **characterised in that** the hole-blocking material comprises more than one unit of the formula (1).

9. Organic electroluminescent device according to one or more of Claims 1 to 8, **characterised in that** the glass transition temperature of the compounds of the formula (1) is > 100°C.

10. Organic electroluminescent device according to one or more of Claims 1 to 9, **characterised in that** the layer thickness of the hole-blocking layer is 1 to 50 nm.

11. Organic electroluminescent device according to one or more of Claims 1 to 10, **characterised in that** the matrix for the phosphorescent emitter is selected from the classes of the carbazoles, ketones, imines, phosphine oxides, phosphine sulfides, phosphine selenides, phosphazenes, sulfones, sulfoxides, silanes, polypodal metal complexes or oligophenylenes based on spirobifluorenes.

12. Organic electroluminescent device according to one or more of Claims 1 to 11, **characterised in that** the phosphorescent emitter is a compound which contains at least one element having an atomic number of greater than 36 and less than 84.

13. Organic electroluminescent device according to Claim 12, **characterised in that** the phosphorescent emitter contains at least one element selected from the elements molybdenum, tungsten, rhenium, ruthenium, osmium, rhodium, iridium, palladium, platinum, silver, gold and europium.

14. Organic electroluminescent device according to one or more of Claims 1 to 13, **characterised in that** one or more layers are applied by a sublimation process.

15. Organic electroluminescent device according to one or more of Claims 1 to 13, **characterised in that** one or more layers are applied by the OVPD (organic vapour phase deposition) process or with the aid of carrier-gas sublimation.

16. Organic electroluminescent device according to one or more of Claims 1 to 13, **characterised in that** one or more layers are applied by a printing process.

17. Use of the design of the electronic devices according to one or more of Claims 1 to 13 for organic transistors, organic integrated circuits, organic solar cells, organic laser diodes or photoreceptors.

18. Compounds of the formula (2) comprising at least one 9,9'-spirobifluorene unit, **characterised in that** at least one triazine unit is bonded to the 9,9'-spirobifluorene, 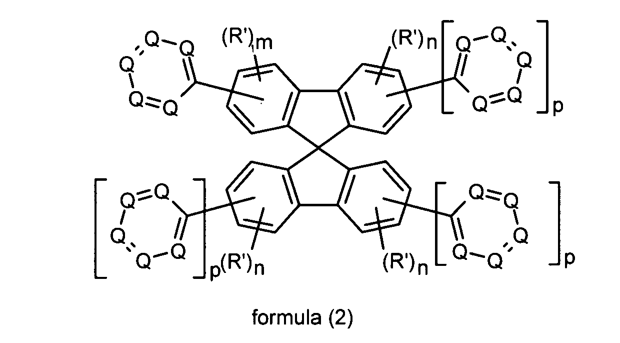 where R and R¹ have the same meaning as defined in Claim 1, and the other symbols and indices have the following meaning:
Q is on each occurrence, identically or differently, N or CR, with the proviso that three Q stand for nitrogen and two Q stand for CR;
R' is on each occurrence, identically or differently, R or F, Cl, Br, I, B(R¹)₂ or B(OR¹)₂;
m is on each occurrence, identically or differently, 0, 1, 2 or 3;
n is on each occurrence, identically or differently, 0, 1, 2, 3 or 4, with the proviso that n must not be 4 if p = 1;
p is on each occurrence, identically or differently, 0 or 1;
the following compound is excluded here:

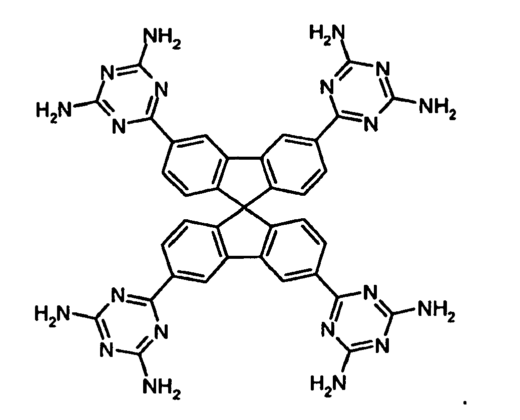

19. Compounds according to Claim 18, **characterised in that** 1,2,4-triazine or 1,3,5-triazine is involved.

20. Compounds according to Claim 18 or 19, **characterised in that** the following applies to the symbols and indices:
R is on each occurrence, identically or differently, an aromatic or heteroaromatic ring system having 1 to 10 aromatic C atoms, which may be substituted by one or more non-aromatic radicals R, where a plurality of substituents R, both on the same ring and also on different rings, may together in turn form a further mono- or polycyclic, aliphatic or aromatic ring system;
R' is on each occurrence, identically or differently, R, a straight-chain, branched or cyclic alkyl group having 1 to 10 C atoms, in which one or more non-adjacent CH₂ groups may be replaced by -R¹C=CR¹-, -C≡C-, Si(R¹)₂, Ge(R¹)₂, Sn(R¹)₂, -O-, -S- or -NR¹-, or Br, I or B(OR¹)₂;
m is on each occurrence equal to 0;
n is on each occurrence, identically or differently, 0 or 1;
the other symbols and indices are as defined above under formulae (1) and (2).

21. Compounds according to one or more of Claims 18 to 20, **characterised in that** two triazine units are present, both bonded to the same fluorene sub-unit of the spirobifluorene.

22. Polymers or dendrimers comprising one or more compounds according to one or more of Claims 18 to 21.

23. Use of compounds, polymers or dendrimers according to one or more of Claims 18 to 22 in electronic devices.

24. Electronic device comprising at least one compound, polymer or dendrimer according to one or more of Claims 18 to 23.

25. Electronic device according to Claim 24, **characterised in that** it is an organic light-emitting diode, an organic solar cell, an organic transistor, an organic integrated circuit, an organic laser diode or an organic photoreceptor.

## Revendications

1. Dispositif électroluminescent organique comprenant une anode, une cathode et une couche d'émission, constituée d'au moins un matériau de matrice qui est dopé avec au moins un émetteur phosphorescent, **caractérisé en ce qu'**une couche de blocage de trous qui comprend un composé de la formule (1) 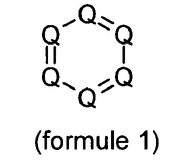 dans laquelle ce qui suit s'applique aux symboles et indices utilisés:
Q est à chaque occurrence, de façon identique ou différente, N ou CR, sous réserve qu'au moins deux et un maximum de quatre Q représentent azote;
R est à chaque occurrence, de façon identique ou différente, H, NO₂, CN, N(R¹)₂, un groupe alkyle ou alcoxy en chaîne droite, ramifié ou cyclique ayant 1 à 40 atomes de C, dans lequel un ou plusieurs groupes CH₂ non adjacents peuvent être remplacés par -R¹C=CR¹-, -C≡C-, Si(R¹)₂, Ge(R¹)₂, Sn(R¹)₂, -O-, -S- ou -NR¹- et dans lequel un ou plusieurs atomes de H peuvent être remplacés par F ou un groupe aromatique R¹, ou
un système de cycle aromatique ou hétéroaromatique ou un groupe aryloxy ou hétéroaryloxy, chacun ayant 1 à 40 atomes de C aromatiques, dans lequel un ou plusieurs atomes de H peuvent être remplacés par F, Cl, Br ou I ou qui peuvent être substitués par un ou plusieurs radicaux non aromatiques R; une pluralité de substituants R peuvent ici former un système de cycle supplémentaire aliphatique ou aromatique mono- ou polycyclique;
ou un système de cycle aromatique ou hétéroaromatique, qui est lié via un groupe divalent -Z-, ou un groupe aryloxy ou hétéroaryloxy, chacun ayant 1 à 40 atomes de C aromatiques, dans lequel un ou plusieurs atomes de H peuvent être remplacés par F, Cl, Br ou I ou qui peuvent être substitués par un ou plusieurs radicaux non aromatiques R; une pluralité de substituants R peuvent ici former un système de cycle supplémentaire aliphatique ou aromatique mono- ou polycyclique;
R¹ est à chaque occurrence, de façon identique ou différente, H ou un radical hydrocarbure aliphatique, aromatique ou hétéroaromatique ayant 1 à 20 atomes de C, dans lequel une pluralité de substituants R¹ ou R¹ avec R peuvent également former un système de cycle supplémentaire aliphatique ou aromatique mono- ou polycyclique;
Z est à chaque occurrence, de façon identique ou différente, un radical en chaîne droite, ramifié ou cyclique, de préférence conjugué, ayant 1 à 40 atomes de C, qui est de préférence en conjugaison avec les deux autres substituants, où le nombre d'atomes dans Z qui lient le groupe de la formule (1) et le radical aromatique est de préférence un nombre pair, où un ou plusieurs atomes de C non adjacents peuvent être remplacés par -O-, -S- ou -NR¹- ou un ou plusieurs atomes de C peuvent être substitués par un radical R¹ ou halogène;
sous condition que R ne contienne par phénylpyridine substituée ou non substituée;
**caractérisé en ce qu'**un dérivé 9,9'-spirobifluorène, un dérivé fluorène 9,9-di-substitué, un dérivé indénofluorène 6,6- et/ou 12,12-di- ou tétrasubstitué, un dérivé tétraarylméthane ou un dérivé triptycène est présent dans au moins un des radicaux R,
est incorporée entre la couche d'émission et la cathode.

2. Dispositif électroluminescent organique selon la revendication 1, **caractérisé en ce qu'**un dérivé 9,9'-spirobifluorène est présent dans au moins un des radicaux R dans des composés de la formule (1).

3. Dispositif électroluminescent organique selon la revendication 1 ou 2, **caractérisé en ce qu'**une couche d'injection de trous et/ou une couche de transport de trous et/ou une couche d'injection d'électrons et/ou une couche de transport d'électrons est présente.

4. Dispositif électroluminescent organique selon une ou plusieurs des revendications 1 à 3, **caractérisé en ce que** la couche de blocage de trous comprend au moins 50% de composés de la formule (1).

5. Dispositif électroluminescent organique selon la revendication 4, **caractérisé en ce que** la couche de blocage de trous est constituée seulement de composés de la formule (1).

6. Dispositif électroluminescent organique selon une ou plusieurs des revendications 1 à 5, **caractérisé en ce que** les structures de la formule (1) sont choisies parmi les groupes des pyridazines, pyrimidines, pyrazines, 1,2,3-, 1,2,4- ou 1,3,5-triazines.

7. Dispositif électroluminescent organique selon la revendication 6, **caractérisé en ce que** les structures de la formule (1) sont choisies parmi les groupes des 1,2,4-triazines ou 1,3,5-triazines.

8. Dispositif électroluminescent organique selon une ou plusieurs des revendications 1 à 7, **caractérisé en ce que** le matériau de blocage de trous comprend plus d'une unité de la formule (1).

9. Dispositif électroluminescent organique selon une ou plusieurs des revendications 1 à 8, **caractérisé en ce que** la température de transition vitreuse des composés de la formule (1) est > 100°C.

10. Dispositif électroluminescent organique selon une ou plusieurs des revendications 1 à 9, **caractérisé en que** l'épaisseur de couche de la couche de blocage de trous est de 1 à 50 nm.

11. Dispositif électroluminescent organique selon une ou plusieurs des revendications 1 à 10, **caractérisé en que** la matrice de l'émetteur phosphorescent est choisie parmi les classes des carbazoles, cétones, imines, oxydes de phosphine, sulfures de phosphine, séléniures de phosphine, phosphazènes, sulfones, sulfoxydes, silanes, complexes de métal polypodal ou oligophénylènes basés sur spirobifluorènes.

12. Dispositif électroluminescent organique selon une ou plusieurs des revendications 1 à 11, **caractérisé en ce que** l'émetteur phosphorescent est un composé qui contient au moins un élément ayant un numéro atomique supérieur à 36 et inférieur à 84.

13. Dispositif électroluminescent organique selon la revendication 12, **caractérisé en ce que** l'émetteur phosphorescent contient au moins un élément choisi parmi les éléments molybdène, tungstène, rhénium, ruthénium, osmium, rhodium, iridium, palladium, platine, argent, or et europium.

14. Dispositif électroluminescent organique selon une ou plusieurs des revendications 1 à 13, **caractérisé en ce qu'**une ou plusieurs couches sont appliquées par un procédé de sublimation.

15. Dispositif électroluminescent organique selon une ou plusieurs des revendications 1 à 13, **caractérisé en ce qu'**une ou plusieurs couches sont appliquées par le procédé OVPD (dépôt organique en phase vapeur) ou à l'aide de sublimation de gaz porteur.

16. Dispositif électroluminescent organique selon une ou plusieurs des revendications 1 à 13, **caractérisé en ce qu'**une ou plusieurs couches sont appliquées par un procédé d'impression.

17. Utilisation de la conception des dispositifs électroniques selon une ou plusieurs des revendications 1 à 13 pour des transistors organiques, des circuits intégrés organiques, des cellules solaires organiques, des diodes laser ou photorécepteurs organiques.

18. Composés de la formule (2) comprenant au moins une unité 9,9'-spirobifluorène, **caractérisés en ce qu'**au moins une unité triazine est liée au 9,9'-spirobifluorène, 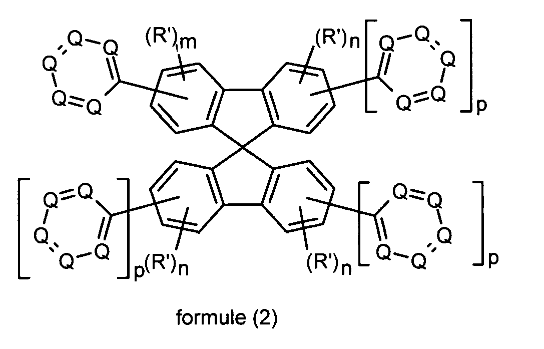 où R et R¹ ont la même signification que celle définie dans la revendication 1, et les autres symboles et indices ont la signification suivante:
Q est à chaque occurrence, de façon identique ou différente, N ou CR, sous réserve que trois Q représentent azote et deux Q représentent CR;
R' est à chaque occurrence, de façon identique ou différente, R ou F, CI, Br, I, B(R¹)₂ ou B(OR¹)₂;
m est à chaque occurrence, de façon identique ou différente, 0, 1, 2 ou 3;
n est à chaque occurrence, de façon identique ou différente, 0, 1, 2, 3 ou 4, sous réserve que n ne soit pas 4 si p = 1;
p est à chaque occurrence, de façon identique ou différente, 0 ou 1;
le composé suivant est exclu ici:

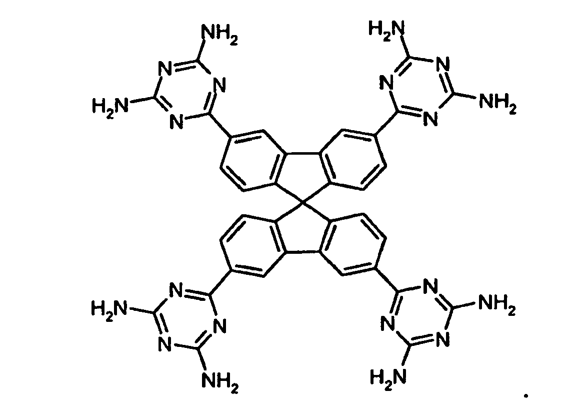

19. Composés selon la revendication 18, **caractérisés en ce que** 1,2,4-triazine ou 1,3,5-triazine est mise en jeu.

20. Composés selon la revendication 18 ou 19, **caractérisés en ce que** ce qui suit s'applique aux symboles et indices:
R est à chaque occurrence, de façon identique ou différente, un système de cycle aromatique ou hétéroaromatique ayant 1 à 10 atomes de C aromatiques, qui peuvent être substitués par un ou plusieurs radicaux non aromatiques R, où une pluralité de substituants R, à la fois sur le même cycle ou sur des cycles différents, peuvent ensemble à leur tour former un système de cycle supplémentaire aliphatique ou aromatique mono- ou polycyclique;
R' est à chaque occurrence, de façon identique ou différente, R, un groupe alkyle en chaîne droite, ramifié ou cyclique ayant 1 à 10 atomes de C, dans lequel un ou plusieurs groupes CH₂ non adjacents peuvent être remplacés par -R¹C=CR¹-, -C≡C-, Si(R¹)₂, Ge(R¹)₂, Sn(R¹)₂, -O-, -S- ou -NR¹-, ou Br, I ou B(OR¹)₂;
m est à chaque occurrence égal à 0;
n est à chaque occurrence, de façon identique ou différente, 0 ou 1;
les autres symboles et indices sont comme défini ci-dessus sous les formules (1) et (2).

21. Composés selon une ou plusieurs des revendications 18 à 20, **caractérisés en ce que** deux unités triazine sont présentes, les deux sont liées à la même sous-unité fluorène du spirobifluorène.

22. Polymères ou dendrimères comprenant un ou plusieurs composés selon une ou plusieurs des revendications 18 à 21.

23. Utilisation de composés, polymères ou dendrimères selon une ou plusieurs des revendications 18 à 22 dans des dispositifs électroniques.

24. Dispositif électronique comprenant au moins un composé, polymère ou dendrimère selon une ou plusieurs des revendications 18 à 23.

25. Dispositif électronique selon la revendication 24, **caractérisé en ce qu'**il est une diode émettrice de lumière organique, une cellule solaire organique, un transistor organique, un circuit intégré organique, une diode laser organique ou un photorécepteur organique.
